# EUROPEAN PATENT APPLICATION

(11) **EP 4 578 949 A1**
(43) Date of publication of application: **02.07.2025**
(21) Application number: 23857347.1
(22) Date of filing: 22.08.2023
(51) Int. Cl.: C12N 15/29, C12Q 1/6851, C12Q 1/6869, A01G 7/00, A01H 6/46

(54) **ULTRA-CHASMOGAMOUS WHEAT, METHOD FOR PRODUCING ULTRA-CHASMOGAMOUS WHEAT, AND METHOD FOR EVALUATING ULTRA-CHASMOGAMOUS WHEAT**

(30) Priority: 22.08.2022 JP 2022131955
(71) Applicant: Okayama University, Okayama-shi, Okayama 700-8530 (JP); National University Corporation Tottori University, Tottori-shi Tottori 680-8550 (JP)
(72) Inventor: KOMATSUDA Takao, Tsukuba-shi Ibaraki 3058518 (JP); OONO Youko, Tsukuba-shi Ibaraki 3058518 (JP); SATO Kazuhiro, Okayama-shi Okayama 7008530 (JP); HISANO Hiroshi, Okayama-shi Okayama 7008530 (JP); SAKUMA Shun, Tottori-shi Tottori 6808550 (JP)
(74) Representative: J A Kemp LLP
(86) International application number: PCT/JP2023/030178
(87) International publication number: WO 2024/043238

(57) **Abstract**

It was found that the flowering ability of a *Triticeae* plant could be improved by suppressing the function of the Sof1 gene, and that the flowering ability of a *Triticeae* plant can be evaluated based on suppression of the function of the Sof1 gene.

## Description

### [Technical Field]

The present invention relates to a *Triticeae* plant with improved flowering ability, and its production and evaluation method, which targets Sof1 gene.

### [Background Art]

The global supply of grains may become strained by a variety of factors, including the increasing food demand due to population growth particularly in developing countries as well as the decreased production due to frequent occurrence of extreme weather events and water resource constraints. As one means for solving this problem, the development of new varieties of grains that can increase agricultural productivity is desired.

Heterosis is the phenomenon in which the productivity of individuals of the first filial generation (F1 generation) exceeds that of the parents, and it has been utilized in the development of new varieties of various crops. Although the heterosis effect is prominent in barley, which has extremely high genetic diversity, the heterosis technique has not matured due to the low productivity of hybrid seeds from cross-pollination, as barley is inherently self-pollinating.

Efforts have been made to identify genes that regulate the flowering ability in grains in order to improve the efficiency of cross-pollination. For example, the small lodicules of cleistogamous barley varieties are regulated by a single recessive gene, cleistogamy 1 (clyl), located on the long arm of the chromosome 2H (Non-patent Literature 1). This gene has been reported to be an ortholog of the AP2 transcription factor in *Arabidopsis thaliana* (Non-patent Literature 2). The chasmogamous Cly1 gene (Cly1.a) in barley increases the width and thickness of the lodicules, pushes the lemma and palea outward from the floret, and mechanically promotes flowering (Non-patent Literature 2). The difference between cleistogamous Cly1.b and chasmogamous Cly1.a is caused by a single nucleotide substitution within the microRNA172 (miR172) specific binding site in the sequence coding clyl. In wild type varieties having Cly1.a, the cleavage of clyl transcripts mediated by miR172 is reduced. As a result, the translated CLY1 protein accumulates at high levels, allowing the suppressed lodicules to develop normally (Non-patent Literatures 2, 3).

By utilizing chasmogamous Cly1.a, the chasmogamous trait can be imparted to the cleistogamous varieties. However, the flowering ability imparted by this gene alone is insufficient to improve the productivity of hybrid seeds through cross-pollination, and a dramatic improvement in the flowering ability is still desired.

### [Citation List]

### [Non Patent Literature]

[NPL 1] Turuspekov, Y. et al., (2004) Theor. Appl. Genet., 109:480-487.
[NPL 2] Nair, S. K. et al., (2010) Proceedings of the National Academy of Sciences, 107 (1):490-495.
[NPL 3] Anwar, N. et al., (2018) Annals of Botany, 122(2) :251-265.

### [Summary of Invention]

### [Technical Problem]

The present invention has been made in view of the problems associated with the above prior art, and an object of the present invention is to provide a *Triticeae* plant with a dramatically improved flowering ability, as well as its production and evaluation method.

### [Solution to Problem]

In order to attain the above object, the present inventors first conducted screening for mutants with improved flowering ability by irradiating a chasmogamous wild barley strain (OUH602) with gamma rays. As a result, the present inventors successfully screened mutants displaying the super-flowering trait with dramatic improvement in flowering ability. Next, the screened mutants were crossbred with a chasmogamous cultivar (Morex), and map-based cloning was performed using the F2 population. As a result, it was found that the gene responsible for the super-flowering trait is present between the markers HM7H234200 and HM7H246100 on the chromosome 7H, and that this trait is governed by a single recessive gene (this gene governing the super-flowering trait was named "sofl"). It was further found that 43 annotated genes and 5 unannotated pseudogenes are present within this approximately 3.1 Mb candidate region.

Next, in order to investigate the polymorphisms of these genes, whole-genome shotgun sequencing was performed on the mutants, and the genome sequence was compared with that of the wild-type barley strain (OUH602). As a result, three homozygous polymorphisms were detected within the targeted region, two of which were 1 bp insertions located in intergenic regions, while the remaining one was a 22bp deletion located within the Horvu_MOREX_7H01G238100 gene.

To substantiate that this gene is sof1 responsible for the super-flowering trait, mutations were introduced using the CRISPR/Cas9 method, targeting the Sof1 gene (the region near the 22 bp deletion) of the cleistogamous cultivar (Golden Promise). As a result, a mutant (sof1-2) with a 5bp deletion in the Sof1 gene was obtained, which formed lodicules significantly longer than those of Golden Promises. Thus, the effect of the mutation was confirmed. However, this mutant did not form lodicules large enough to exhibit the super-flowering trait, being influenced by the cleistogamous clyl.b gene of Golden Promise. For the purpose of eliminating the influence of the clyl.b gene, the mutant was crossbred with barley varieties Sv73528 and Adorra, which have the chasmogamous Cly1.a gene. As a result, the lodicules of the obtained individuals were significantly longer than those of Sv73528 and Adorra, exhibiting the super-flowering trait. Based on the above, it has been determined that sof1 gene is the gene responsible for the super-flowering trait. Furthermore, the present inventors searched for homologous genes in other plants, and as a result, found that a gene corresponding to the barley gene is present also in wheat. The present inventors also succeeded in obtaining wheat mutants exhibiting the super-flowering trait.

Based on the above, the present inventors have concluded that the flowering ability of a *Triticeae* plant can be enhanced by suppressing the function of Sof1 gene (the wild-type gene corresponding to the mutant sof1 gene) and that the flowering ability of a *Triticeae* plant can be evaluated based on the suppression of Sof1 gene function, leading to the completion of the present invention.

The present invention more specifically includes the following aspects.
[1] A method for producing a *Triticeae* plant with improved flowering ability, which comprises artificially suppressing the function of the following endogenous gene (a) or (b) in a *Triticeae* plant:
   (a) an endogenous gene encoding a protein consisting of an amino acid sequence of SEQ ID NOs: 2, 5, 8 or 11;
   (b) an endogenous gene encoding a protein consisting of an amino acid sequence having at least 80% homology to the amino acid sequence of SEQ ID NOs: 2, 5, 8 or 11.
[2] A *Triticeae* plant with improved flowering ability, wherein the function of the following endogenous gene (a) or (b) is artificially suppressed:
   (a) an endogenous gene encoding a protein consisting of an amino acid sequence of SEQ ID NOs: 2, 5, 8 or 11;
   (b) an endogenous gene encoding a protein consisting of an amino acid sequence having at least 80% homology to the amino acid sequence of SEQ ID NOs: 2, 5, 8 or 11.
[3] A method for evaluating flowering ability of a *Triticeae* plant, which comprises analyzing a base sequence of the following endogenous gene (a) or (b) or a regulatory region thereof in a *Triticeae* plant:
   (a) an endogenous gene encoding a protein consisting of an amino acid sequence of SEQ ID NOs: 2, 5, 8 or 11;
   (b) an endogenous gene encoding a protein consisting of an amino acid sequence having at least 80% homology to the amino acid sequence of SEQ ID NOs: 2, 5, 8 or 11.
[4] A method for evaluating flowering ability of a *Triticeae* plant, which comprises analyzing the expression of the following endogenous gene (a) or (b) in a *Triticeae* plant:
   (a) an endogenous gene encoding a protein consisting of an amino acid sequence of SEQ ID NOs: 2, 5, 8 or 11;
   (b) an endogenous gene encoding a protein consisting of an amino acid sequence having at least 80% homology to the amino acid sequence of SEQ ID NOs: 2, 5, 8 or 11.
[5] A method for producing a *Triticeae* plant with improved flowering ability, which comprises crossbreeding the *Triticeae* plant of claim 2 with another arbitrary *Triticeae* plant.

### [Advantageous Effects of Invention]

According to the present invention, a *Triticeae* plant with dramatically improved flowering ability can be produced. This *Triticeae* plant exhibits a large flowering angle due to the significant expansion of its lodicules. This trait makes dispersal of pollen easier when introduced into a pollen parent in barley breeding, while makes pollination easier when introduced into a male-sterile seed parent. This enables dramatic improvement in the hybrid seed productivity through cross-pollination.

### [Brief Description of Drawings]

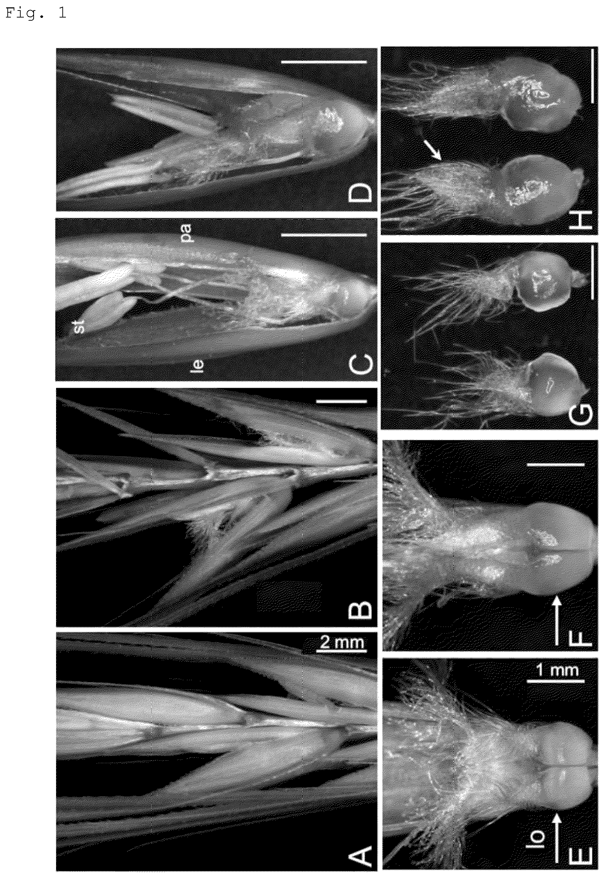

[Fig. 1] Photographs of the wild-type OUH602 (WT) and the mutant 44205 (MT). Spikelet of (A) the wild-type OUH602 and (B) the mutant 44205. Lodicules of (C) the wild-type OUH602 and (D) the mutant 44205 at the yellow anther stage just before the flowering. Lodicules of (E) the wild-type OUH602 and (F) the mutant 44205 seen from the front of the spikelet. Lodicules of (G) the wild-type OUH602 and (H) the mutant 44205 seen from the side of the spikelet.

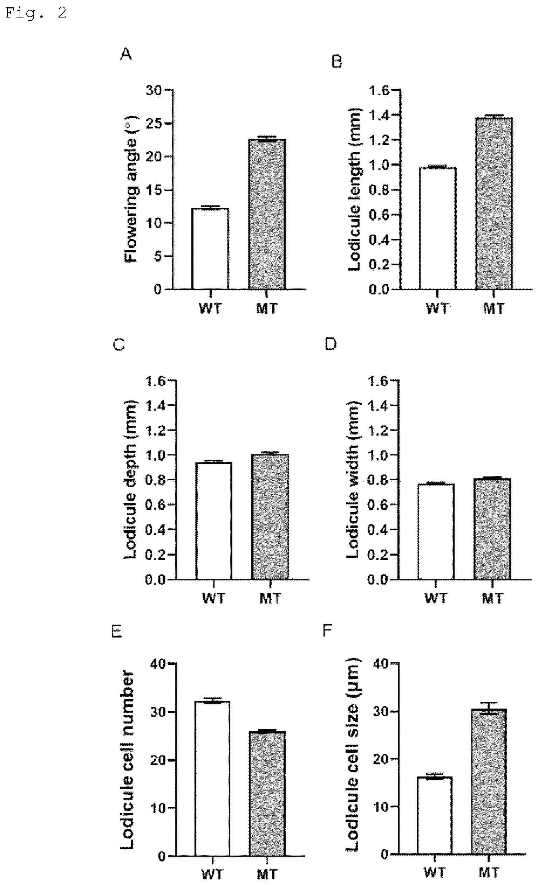

[Fig. 2] Graphs of the wild-type OUH602 (WT) and the mutant 44205 (MT) (continued from Figure 1). (A) Flowering angle, (B) length of lodicule, (C) thickness of lodicule, (D) width of lodicule, (E) number of lodicule cells, (F) length of lodicule cells..

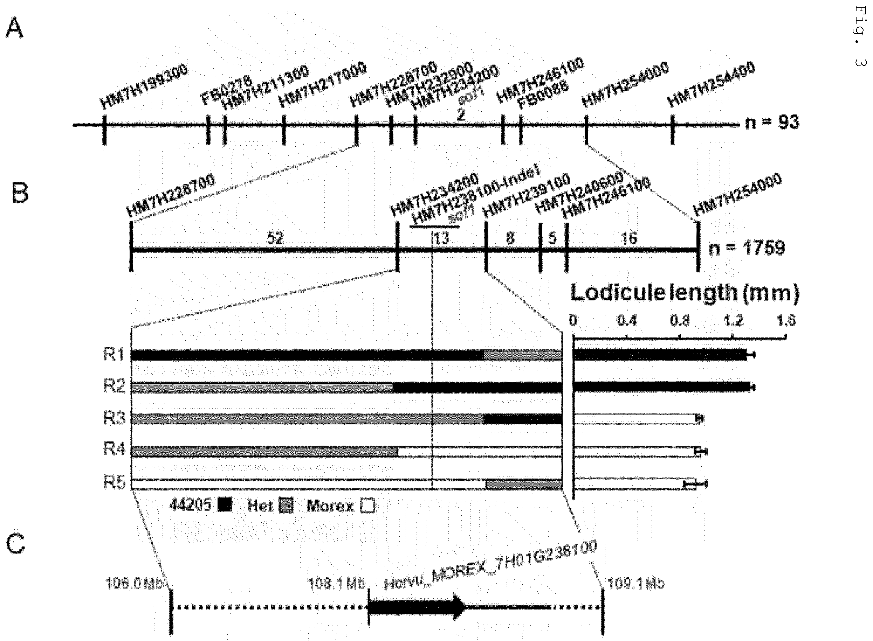

[Fig. 3] Figure showing the map-based cloning of the sof1 gene. (A) High-resolution genetic map of the sof1 gene. In the linkage map, numbers between markers on lines indicate the number of recombinants covered. (B) Fine mapping of the sof1 gene using a large F2 population (n = 1759). (C) Annotated genes with mutations identified by whole genome sequencing in the 3.1 Mb physical region. Arrows indicate the direction of transcription of the annotated genes.

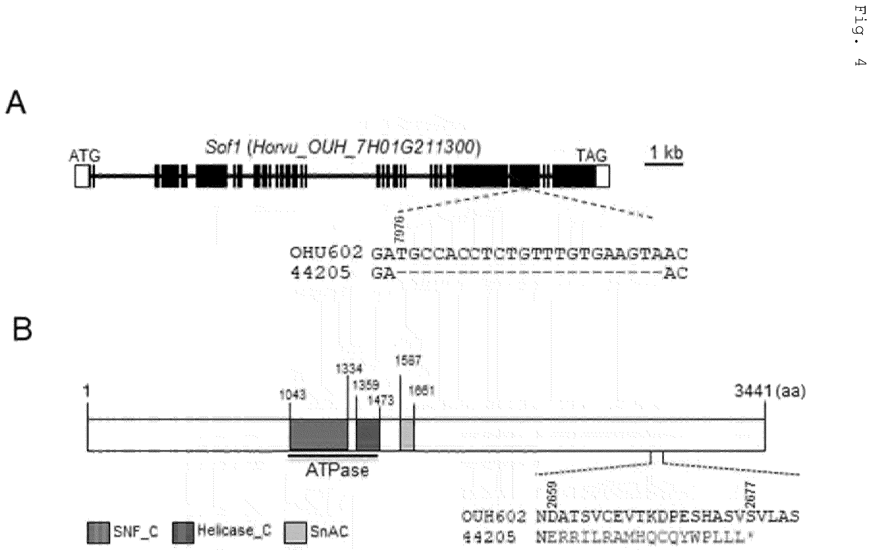

[Fig. 4] Figure showing the map-based cloning of the sof1 gene (continued from Figure 3). (A) Structure of the Sof1 gene. The boxes at both ends represent the 5' and 3' untranslated regions (UTR), the boxes in between represent the coding exons, and the thin lines represent introns. ATG and TAG represent the start codon and stop codon, respectively. The dashed lines represent a 22 bp deletion in the mutant. (B) Structure of the SOF1 protein. Each box (positions 1043-1334, positions 1359-1473, and positions 1587-1661) represents a domain of the SOF1 protein.

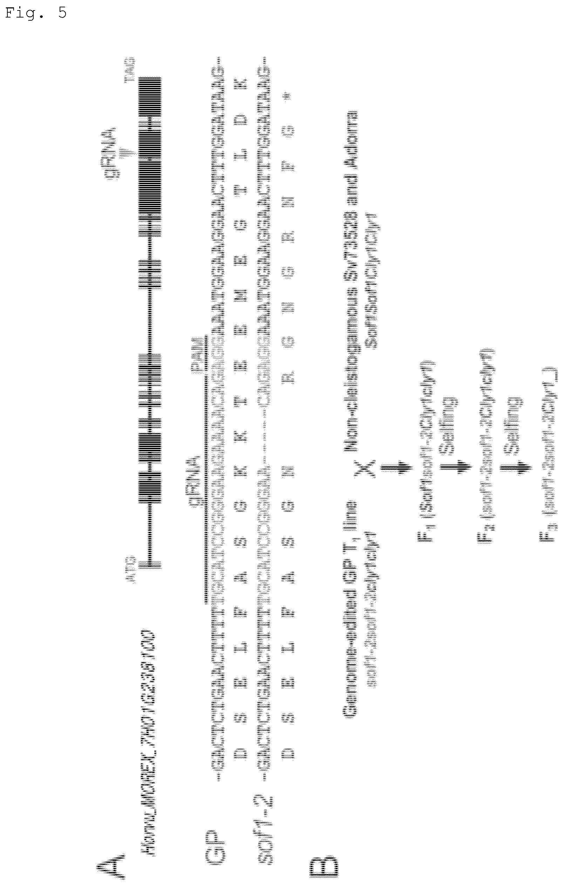

[Fig. 5] Figure showing the characterization of the function of Sof1 through mutation induction based on CRISPR/Cas9. (A) Schematic diagram of the gene structure of the Sof1 candidate gene (Horvu _MOREX_7H01G238100) and the mutant generated by CRISPR/Cas9. Exons and introns are represented by boxes and lines, respectively. Inverted triangle represents the target site of the guide RNA (gRNA) within the gene. The lines above the Golden Promise (GP) sequence represent the target site of gRNA and the protospacer adjacent motif (PAM). Dashed lines represent the deleted nucleotides. (B) Scheme for identification of phenotype in the F3 plants derived from crossbreeding between the edited T1 strain and two chasmogamous barley cultivars. In parentheses, the genotypes of each generation are indicated.

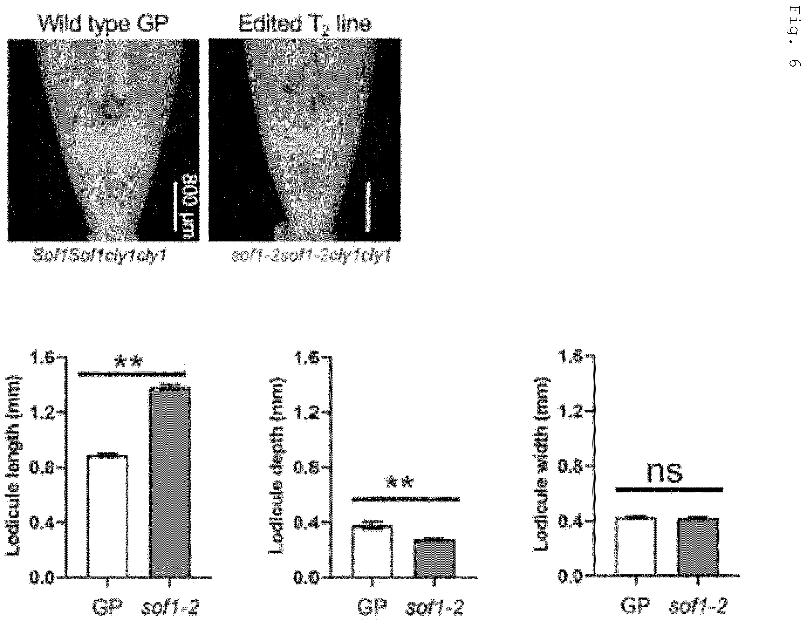

[Fig. 6] Photographs of the wild-type Golden Promise (GP) and the homozygous T2 mutant strain (top) and graphs showing lodicule size (bottom). Each measured value on the graph represents the average of 9-13 lodicules for statistical analysis. Two-tailed Student's t-test. ** represents P < 0.01, and ns represents no significant difference.

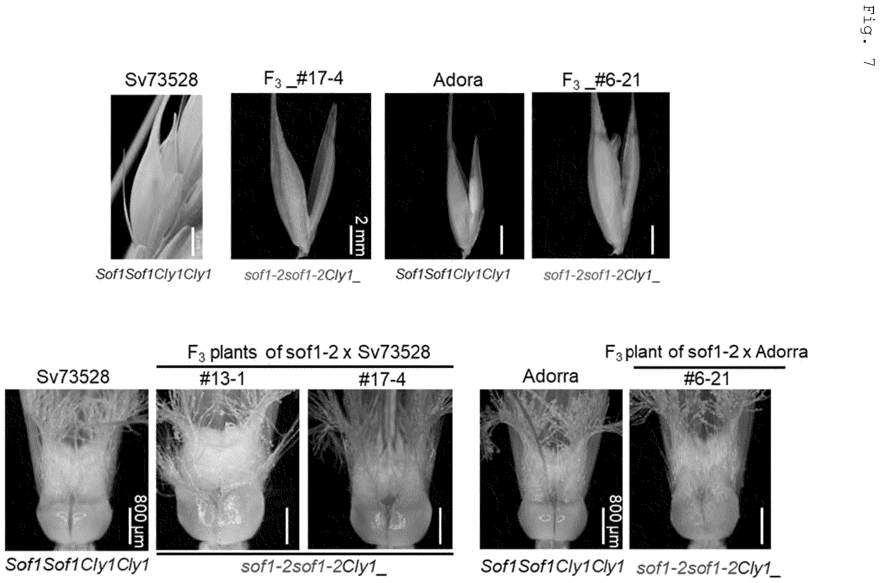

[Fig. 7] Photographs showing a comparison of the flowering angles of florets (top) and lodicule sizes (bottom) in the two chasmogamous cultivars and their corresponding F3 plants.

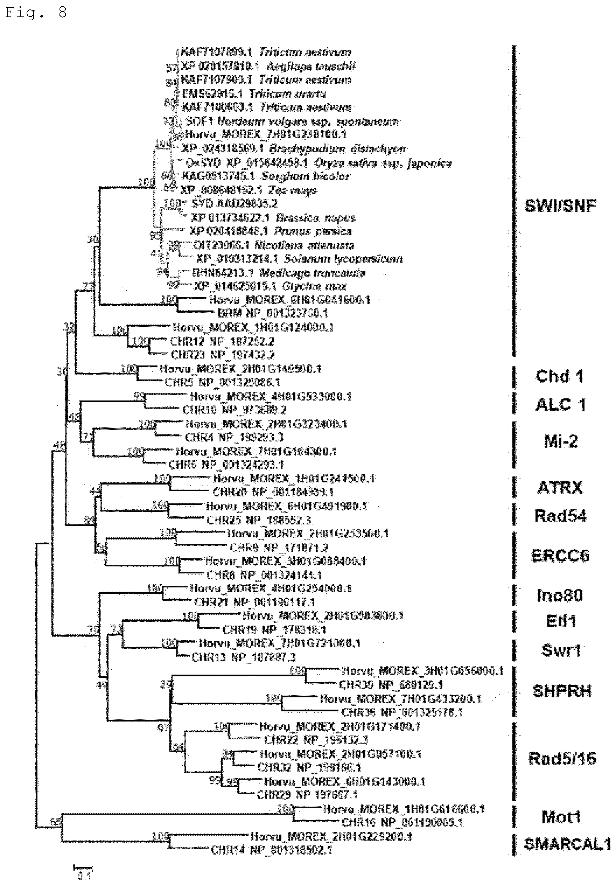

[Fig. 8] Figure showing a phylogenetic analysis of the SOF1 homologs in monocotyledons and dicotyledons. The tree was constructed using the Neighbor-Joining (NJ) method based on the alignment of SOF1 homolog polypeptide sequences. Numbers shown on the branches represent the bootstrapping probabilities.

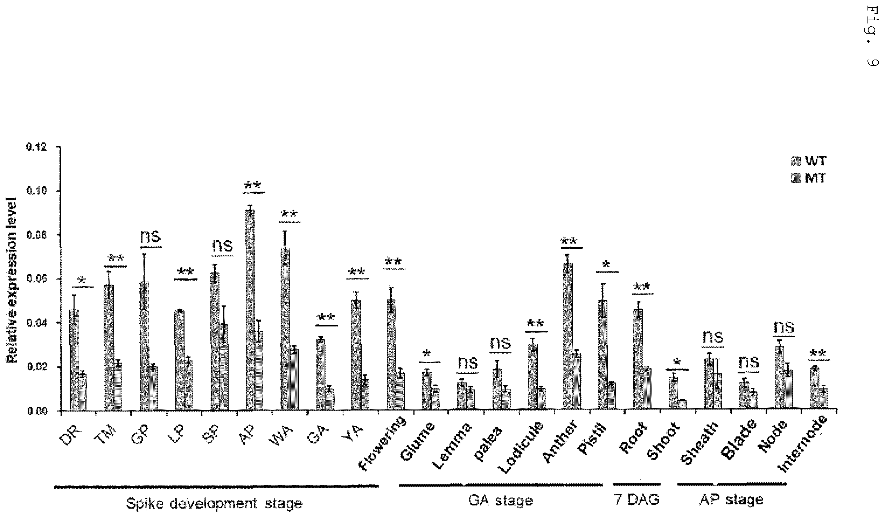

[Fig. 9] Graph showing the amount of Sof1 transcript during the development of the spike, including both vegetative and reproductive organs, in the wild-type OUH602 (WT) and the mutant 44205 (MT). DR (double ridge stage); TM (triple mound stage); GP (glume primordium stage); LP (lemma primordium stage); SP (stamen primordium stage); AP (awn primordium stage); WA (white anther stage); GA (green anther stage); YA (yellow anther stage). DAG (day after germination). Values are presented as mean ± SE (n = 3 independent biological replicates). Statistical significance value was calculated using a Two-tailed Student's t-test. * represents P < 0.05, ** represents P < 0.01, and ns represents no significant difference.

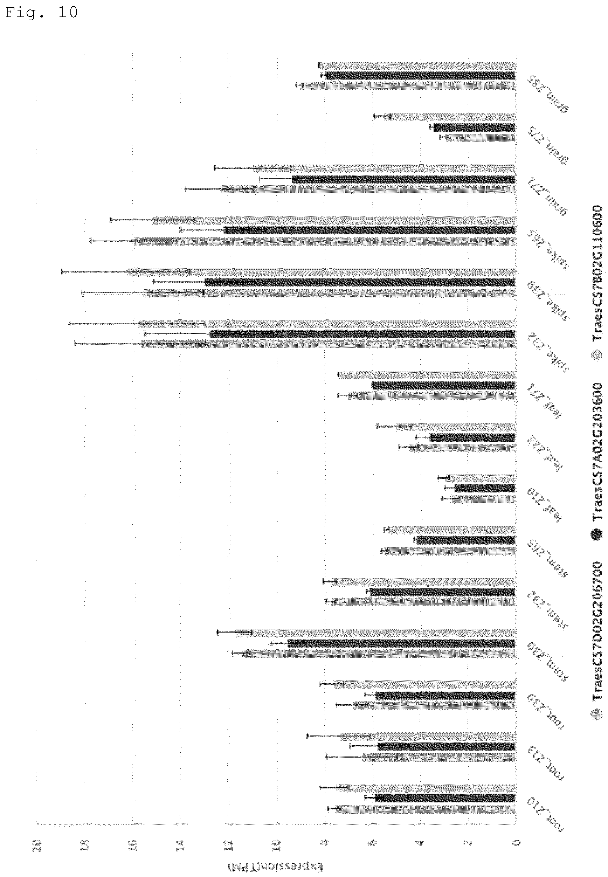

[Fig. 10] Graph showing the amount of Sof1 transcript in the wild-type wheat cultivar (Chinese Spring).

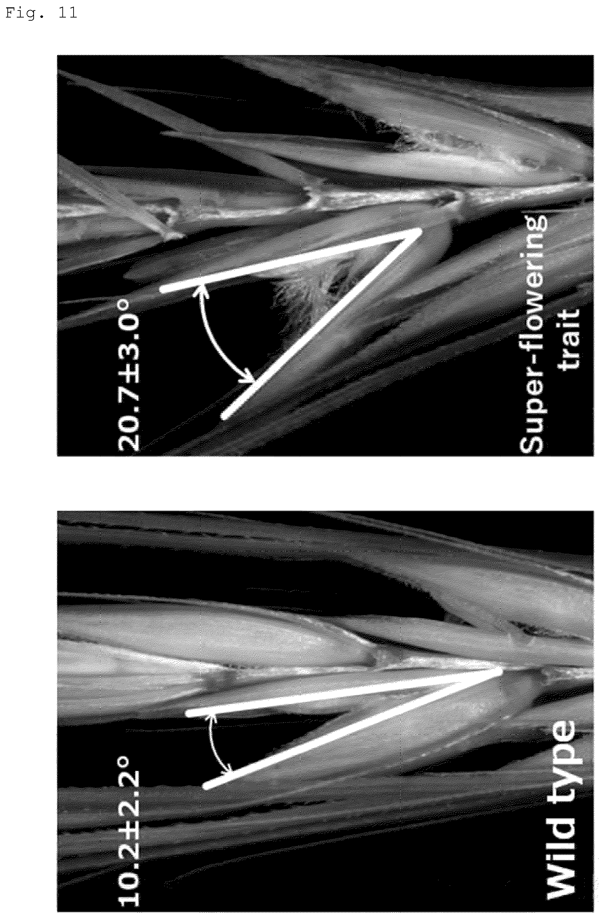

[Fig. 11] Photographs showing the flowering angles of the wild-type OUH602 (left) and the super-flowering mutant of OUH602 (hereinafter referred to as "mutant 44205" unless otherwise specified) (right). The measured flowering angles are shown in the photographs.

### [Description of Embodiments]

### < A method for producing a Triticeae plant with improved flowering ability>

In the present invention, the term "flowering ability" refers to the trait where the flowering angle increases due to the expansion of lodicule. The inflorescence of monocotyledons, such as a *Triticeae* plant, rice and maize, consists of a structure referred to as spikelet and it contains one or more florets. Each floret is composed of reproductive organs (stamens and pistils) and is covered by a pair of organs resembling bracts (palea and lemmas). "Lodicule" is located between these reproductive organs and glumes. Lodicules are generally considered to correspond to petals, and they are present at the base of the reproductive organs (stamens and pistils) on both sides. In case that the lodicules rapidly expand just before flowering, they mechanically separate the palea and lemma, making it easier for pollens to be released from the ruptured anthers, and thereafter the pistil becomes capable of cross-pollination (non-cleistogamy/chasmogamy). In contrast, in case that the lodicules cannot expand, the pollens are concealed within the closed floret, thereby leading to self-pollination (cleistogamy).

In the present invention, the term "improvement of flowering ability" refers to an increase in the flowering angle compared to when the function of the Sof1 gene is not artificially suppressed. The increase in the flowering angle is preferably 2° or more, more preferably 5° or more, further preferably 8° or more, and particularly preferably 10° or more. The flowering angle can be measured as the angle at the intersection of a line along the edge of the lemma (the glume that is farther from the spike axis) and a line along the edge of the palea (the glume that is closer to the spike axis) in the floret of the spike. The flowering angle may vary depending on the species or varieties of a *Triticeae* plant. For example, the flowering angle of the wild-type barley OUH602 is typically 10.2±2.2° (Figure 11 (left)).

In the present invention, the term "a *Triticeae* plant" refers to all plants belonging to the tribe *Triticeae* of the *Paceae* family, which include for example barley (*Hordeum vulgare*)*,* wheat (*Triticum aestivum*)*,* rye (*Secale cereal*)*,* triticale (a hybrid of wheat and rye), and oats (*Avena sativa*)*.* They may be wild-type varieties or cultivated varieties. When a *Triticeae* plant possesses the cleistogamous clyl gene, the effect of suppressing the function of the Sof1 gene may become less pronounced. Therefore, a *Triticeae* plant that will be the target for improving flowering ability is preferably the one that possesses the chasmogamous clyl gene. When suppressing the function of the Sof1 gene in a *Triticeae* plant with the cleistogamous clyl gene, the negative impact of the cleistogamous clyl gene can be eliminated by subsequent crossbreeding with a *Triticeae* plant with the chasmogamous clyl gene, or modifying the clyl gene from the cleistogamous type to the chasmogamous type (e.g., a single base substitution in the specific binding site of miR172) by genome editing, etc.

The method of the present invention comprises artificially suppressing the function of the Sof1 gene (an endogenous gene encoding the SOF1 protein).

The "Sof1 gene" in the present invention belongs to the SWI/SNF subfamily and is assumed to encode a chromatin remodeling ATPase protein. In a *Triticeae* plant expressing the mutant protein, the lodicules expand and the flowering ability is enhanced. Therefore, it is considered that the wild-type protein (SOF1 protein) has the activity of suppressing the expansion of the lodicules in a *Triticeae* plant.

The typical base sequence of cDNA of the Sof1 gene in barley is shown in SEQ ID NO: 1, the base sequence of genomic DNA is shown in SEQ ID NO: 3, and the amino acid sequences of the proteins encoded by these DNAs are shown in SEQ ID NO: 2. Meanwhile, the typical base sequence of cDNA in wheat is shown in SEQ ID NOs: 4, 7, and 10, the base sequence of genomic DNA is shown in SEQ ID NOs: 6, 9, and 12, and the amino acid sequences of the proteins encoded by these DNAs are shown in SEQ ID NOs: 5, 8, and 11.

In nature, there may arise individual differences in nucleotide sequences. With the current level of technology, once a specific gene is obtained, a person ordinarily skilled in the art can identify the corresponding gene in the same or other plant varieties using the nucleotide sequence information of the specific gene. Therefore, these homologous genes are included in the Sof1 gene in the present invention, as long as the suppression of their function results in the improved flowering ability

The amino acid sequence of the protein encoded by the homologous gene typically share a high sequence homology with the amino acid sequence of the protein encoded by the specific gene. Herein, "high sequence homology" means at least 80% or more, preferably 85% or more, more preferably 90% or more, and further preferably 95% or more (e.g., 96% or more, 97% or more, 98% or more, or 99% or more) homology.

The sequence homology can be determined using the BLAST program (Altschul et al. J. Mol. Biol., (1990) 215:403-410). The program is based on the BLAST algorithm developed by Karlin and Altschul (Karlin, S. & Altschul, SF., (1990) Proc. Natl. Acad. Sci. USA, 87:2264-2268; Karlin, S. & Altschul, SF., (1993) Proc. Natl. Acad. Sci. USA, 90:5873-5877). For example, when analyzing an amino acid sequence using the BLAST, the parameters may be set as follows: score = 50, wordlength = 3, for example. When analyzing an amino acid sequence using the Gapped BLAST program, it may be performed as described by Altschul et *al.* (Altschul, SF. et al., (1997) Nucleic Acids Res., 25:3389-3402.). When using the BLAST and Gapped BLAST programs, the default parameters of each program are used. The specific methods for these analyses are known in the art.

These homologous genes can be obtained, for example, by utilizing hybridization techniques (Southern, E. M., (1975) J. Mol. Biol., 98:503) or polymerase chain reaction (PCR) techniques (Saiki, R. K. et al., (1985) Science, 230:1350-1354; Saiki, R. K. et al., (1988) Science, 239:487-491).

The "suppression of the function of the Sof1 gene" in the present invention includes both complete suppression (inhibition) and partial suppression of its function. It also includes both the suppression of the SOF1 protein activity (e.g., the activity for suppressing the expansion of lodicules) and the suppression of the Sof1 gene expression (transcriptional suppression and translational suppression).

The suppression of the function of the Sof1 gene can be achieved, for example, by introducing a mutation into the Sof1 gene or its regulatory region (e.g., the promoter region). The mutation introduced into the Sof1 gene may be any mutation as long as it suppresses its function. Examples of the mutation include a nucleotide substitution, deletion, addition, and/or insertion, while a frame-shift mutation, nonsense mutation, and null mutation are preferred.

Any number of mutations may be introduced into the Sof1 gene, as long as they suppress its function. The number of mutations may be one or multiple (e.g., 2, 3 or fewer, 5 or fewer, 10 or fewer, 20 or fewer, 30 or fewer, 40 or fewer, or 50 or fewer). In fact, it was demonstrated in the examples of the present invention that the nucleotide deletions in the Sof1 gene of barley (the 22-base deletion [sofl mutant] and the 5-base deletion [sofl-2 mutant]) resulted in a mutant protein that was shortened at the C-terminal side due to frameshifting, leading to an improvement in the flowering ability of barley. The SOF1 protein encoded by the Sof1 gene has two functional domains (ATPase domain and SnAC domain) at its N-terminal side. However, the loss of SOF1 protein function can be achieved also through a mutation on the C-terminal side, where these functional domains are absent. Therefore, the mutation introduced into the Sof1 gene does not necessarily need to result in the complete loss of the amino acid sequence encoded by the gene. The mutation may be introduced in the gene such that a portion of the amino acid sequence is lost or altered.

When deleting a portion of the expressed protein through genetic mutation, from the viewpoint of suppressing the SOF1 protein activity, the deletion is preferably 5% or more, more preferably 10% or more, more preferably 20% or more, further preferably 30% or more, further more preferably 50% or more (e.g., 60% or more, 70% or more, 80% or more, 90% or more, or 95% or more) deletion of the total protein.

The introduction of mutations into the Sof1 gene can be achieved by a person ordinarily skilled in the art using methods known in the art. Among the methods known in the art, genome editing method is preferred because of its ability to efficiently introduce site-specific mutations.

The genome editing method is a method for modifying target genes using site-directed nucleases. Examples of site-directed nucleases include Cas nucleases (CRISPR/Cas system), which are imparted with site specificity by forming a complex with guide RNA, transcription-activator-like effector nucleases (TALENs), and zinc finger nucleases (ZFNs). As CRISPR/Cas systems, various systems may be used, including type II system of class 2 (containing Cas9 as the Cas), type V system of class 2 (containing Cas12a (Cpf1), Cas12b (C2c1), Cas12c (C2c3), Cas12d (CasY), Cas12e (CasX), Cas14, etc. as the Cas), type VI system of class 2 (containing Cas13a (C2c2), Cas13b, Cas13c, etc. as the Cas), type I system of class 1 (containing Cas3 as the Cas), etc.

Examples of other methods for introducing mutations into genes include, but not limited to, physical mutation induction methods, methods using chemical mutagens, methods using transposons, etc.

Examples of physical mutation induction methods include gamma-ray radiation, heavy-ion beam (HIB) irradiation, fast neutron irradiation, and ultraviolet irradiation (Hayashi Y. et al. (2007) Cyclotrons and Their Applications, 18th International Conference, 237-239; Kazama Y. et al., (2008) Plant Biotechnology, 25:113-117).

Examples of methods using chemical mutagens include methods treating seeds, etc. with chemical mutagens (Zwar and Chandler, Planta (1995) Vol. 197, pp. 39-48). Any chemical mutagens may be used, but examples include N-methyl-N-nitrosourea (MNU), ethyl methanesulfonate (EMS), N-ethyl-N-nitrosourea (ENU), sodium azide, sodium bisulfite, hydroxylamine, N-methyl-N'-nitro-N-nitrosoguanidine (MNNG), N-methyl-N'-nitrosoguanidine (NTG), O-methylhydroxylamine, nitrous acid, formic acid, and nucleotide analogs.

Examples of methods for introducing transposons into genomic DNA include, for example, inserting transposons such as TOS17 or T-DNA into the plant genomic DNA (Kumar, A. & Hirochika, H., (2001) Trends Plant Sci., 6(3):127-134; Tamara, M. et al., (1999) Trends in Plant Science, 4(3) : 90-96) .

When using these methods, typically, from the population subjected to the mutation induction treatment, individuals with mutations in the Sof1 gene are screened.

A *Triticeae* plant with a mutation introduced into the Sof1 gene may be a heterozygote of the mutated gene. In such a case, heterozygotes can be crossbred with each other to produce F1 plants, and from these F1 plants, homozygotes of the mutated gene can be screened. It has been found that the sof1 gene (mutant gene) responsible for the super-flowering trait in the present invention is a recessive gene. Therefore, in order to improve the flowering ability of a *Triticeae* plant by introducing a mutation into the Sof1 gene, it is preferable that the *Triticeae* plant homozygously carry the mutated Sof1 gene. When the *Triticeae* plant carries an unintended mutation, the unintended mutation may be removed through backcrossing with a wild-type *Triticeae* plant.

When the function of the Sof1 gene is suppressed by suppressing the Sof1 gene expression, not only methods introducing a mutation into the regulatory regions of the Sof1 gene, but also methods targeting the transcripts of the Sof1 gene can be used. Examples of such methods include a method using DNA that encodes dsRNA (double-stranded RNA, such as siRNA) complementary to the transcripts of the Sof1 gene; a method using DNA (antisense DNA) that encodes antisense RNA complementary to the transcripts of the Sof1 gene; and a method using DNA that encodes RNA with ribozyme activity of specifically cleaving the transcripts of the Sof1 gene (ribozyme method), etc.

The suppression of the Sof1 gene expression may also be achieved through nucleotide modifications in epigenetic regulations, which does not involve mutations in nucleotides. Examples of epigenetic regulations include DNA methylation and chemical modifications of histones (such as acetylation, methylation, phosphorylation, ubiquitination, etc.).

In the present invention, the treatment for artificially suppressing the function of the Sof1 gene can be performed on the plant body, organs, tissues, or cells of a *Triticeae* plant, depending on the type of techniques used. Examples of organs, tissues, or cells include seeds, microspores, pollens, immature embryos, leaf sections, suspended culture cells, protoplasts, callus, growth points, young spikes, etc.

When a molecule for artificially suppressing the function of the Sof1 gene is a molecule encoded by DNA (e.g., DNA encoding a site-directed nuclease, DNA encoding a transposon, DNA encoding a double-stranded RNA, DNA encoding an antisense RNA, DNA encoding an RNA with ribozyme activity, etc. as discussed above), it may be introduced into a *Triticeae* plant in the form of a vector in which the DNA is inserted. Any vector may be used, as long as it is capable of expressing the inserted DNA within the cells of a *Triticeae* plant. The vector typically comprises a promoter for constitutive or inducible expression of the DNA. Examples of promoters for constitutive expression include the U3 promoter from barley, the ubiquitin promoter from rice, the 35S promoter from cauliflower mosaic virus, the actin promoter from rice, and the ubiquitin promoter from maize. Examples of promoters for inducible expression include promoters that are expressed by external factors such as treatment with specific chemicals, low temperature, high temperature, drying, ultraviolet irradiation, or infection or invasion by fungi, bacteria, or viruses.

For introducing a molecule for artificially suppressing the function of the Sof1 gene into a *Triticeae* plant, methods that are known by a person ordinarily skilled in the art, such as the Agrobacterium-mediated method (Agrobacterium method), particle bombardment method, polyethylene glycol method, and electroporation method, etc., may be used.

When cells in which the function of the Sof1 gene has been artificially suppressed are obtained by the above methods, a *Triticeae* plant with improved flowering ability can be obtained by regenerating the plant body of the *Triticeae* plant from these cells. Examples of methods for producing transgenic plants body of a *Triticeae* plant include methods described by Tingay et *al.* (Tingay, S. et al., (1997) Plant J., 11:1369-1376.), Murray et *al.* (Murray, F. et al. (2004) Plant Cell Report, 22:397-402.), and Travalla et *al.* (Travalla, S. et al., (2005) Plant Cell Report, 23:780-789.). Meanwhile, methods described by Tabei et *al.* (Edited by TABEI Yutaka., "KEISHITSUTENKAN PROTOCOL [SHOKUBUTSU HEN]" (Transformation Protocols [Plant Edition]), Kagaku-Dojin Publishing Company, INC., published on September 20, 2012) can be used for transformation and regeneration into plant body.

Once a plant body in which the function of the Sof1 gene is artificially suppressed is obtained, offsprings can be obtained from the plant body through sexual or asexual reproduction. Furthermore, mass production of the plant bodies is also possible based on a reproductive material (e.g., seeds, scions, shoots, callus, protoplasts, etc.) obtained from the plant body, its offsprings, or clones. The present invention also provides the plant bodies, offsprings, clones, and reproductive materials of a *Triticeae* plant with improved flowering ability obtained in this manner.

### <A method for evaluating flowering ability of a Triticeae plant>

One aspect of the method of the present invention comprises analyzing a base sequence of the Sof1 gene or its regulatory region in a *Triticeae* plant.

When analyzing a base sequence of the Sof1 gene or its regulatory region, an amplified product obtained by PCR amplification of the Sof1 gene or its regulatory region can be used. When performing PCR, any primers may be used as long as they can specifically amplify the Sof1 gene or its regulatory region. The primers can be appropriately designed based on the sequence information of the Sof1 gene or its regulatory region.

Evaluation of flowering ability may comprise a step of comparing with a "reference base sequence". The "reference base sequence" typically refers to a base sequence of a gene encoding the amino acid sequences of SEQ ID NO:2 (barley) or the amino acid sequence of SEQ ID NOs: 5, 8, or 11 (wheat), or their regulatory regions.

By comparing the base sequence of the Sof1 gene or its regulatory region in the test *Triticeae* plant with the reference base sequence, it is possible to evaluate whether flowering ability is improved in the test *Triticeae* plant or not. For example, when there is a difference in the base sequence when compared to the reference base sequence (particularly, when there is a significant change in the molecular weight or amino acid sequence of the encoded protein due to occurrence of a new stop codon or a frameshift), the test *Triticeae* plant is evaluated as being highly likely to have the improved flowering ability.

The preparation of DNA for base sequence analysis can be performed by conventional methods, such as the CTAB method. As a *Triticeae* plant used for the preparation of DNA, not only mature plant bodies but also seeds and young plant bodies can be used. Determination of base sequence can be performed by conventional methods, such as the dideoxy method or the Maxam-Gilbert method. Commercially available sequencing kits and sequencers can be used for determination of base sequence.

Whether the base sequence of the Sof1 gene or its regulatory region in the test *Triticeae* plant differs from the reference base sequence or not can also be analyzed indirectly by methods known in the art, such as the PCR-SSCP (single-strand conformation polymorphism) method, apart from directly determining the base sequence as discussed above.

Another aspect of the method of the present invention comprises analyzing the expression of the Sof1 gene.

Herein, "analysis of the expression of gene" includes analysis of the presence or absence and the level of the expression (transcription and translation), as well as analysis of the molecular weight of the expressed product.

The detection of the Sof1 gene at the transcription level can be performed by conventional methods, such as the RT-PCR method, Northern blotting method, or RNA-seq method. The primers used for performing the PCR may be any primer as long as they can specifically amplify the DNA of interest for detection in the present invention, and the primers can be appropriately designed based on the already determined sequence information of the Sof1 gene. Meanwhile, the detection at the translation level can be performed by conventional methods, such as the western blotting method. The antibodies used in the western blotting may be polyclonal antibodies and/or monoclonal antibodies. The methods for preparing these antibodies are well-known to a person ordinarily skilled in the art.

If the result of the detection of gene expression shows that the expression level of the Sof1 gene in the test *Triticeae* plant is significantly lower (e.g., if the Sof1 gene is substantially not expressed) than the expression level in a variety possessing the wild-type Sof1 gene (e.g., OUH602 for barley, Chinese Spring for wheat), it is evaluated that the test *Triticeae* plant is highly likely to be a *Triticeae* plant with the improved flowering ability. Meanwhile, if the molecular weight of the amplified product or expressed product of the Sof1 gene significantly differs from the molecular weight of the expressed product of the wild-type Sof1 gene, it is evaluated that the test *Triticeae* plant is highly likely to be a *Triticeae* plant with the improved flowering ability.

### <A method for producing a Triticeae plant with improved flowering ability>

The present invention provides a method for producing a *Triticeae* plant with improved flowering ability.

The method of the present invention comprises crossbreeding the above *Triticeae* plant with improved flowering ability with another arbitrary *Triticeae* plant.

Examples of "another arbitrary *Triticeae* plant" to be crossbred with the *Triticeae* plant with improved flowering ability include, but not limited to, a *Triticeae* variety in which the function of the Sof1 gene is not suppressed and does not exhibit the super-flowering trait. From the individuals obtained by crossbreeding, a *Triticeae* plant in which the function of the Sof1 gene is suppressed can be screened using the above discussed methods.

In accordance with the method of the present invention, it becomes possible to screen and select varieties of a *Triticeae* plant with improved flowering ability at an early stage, such as during the seeds or young plant stage, enabling the breeding of varieties with this trait in a short period of time.

### [Examples]

Below, the present invention is described more specifically based on the examples. However, the present invention is not limited to the examples provided below.

### A. Materials and Methods

### (1) Plant Materials

The wild-type cleistogamous barley strain (*Hordeum vulgare* subsp. *spontaneum*) OUH602 was obtained from Institute of Plant Science and Resources of the Okayama University. OUH602 was treated with a low-dose cobalt-60 (⁶⁰Co) gamma-ray irradiation (0.24-0.77 Gy per day, irradiation for five days per week) during its growing process from seedlings. Based on the super-flowering phenotype, an OUH602 mutant (strain number: 44205) was screened from the M3 strains derived from 1600 M2 individuals. For genetic analysis, the OUH602 mutant was crossbred with the cultivar "Morex" and a mapping population is produced. Plant cultivation was carried out in a net house (with natural light and unregulated temperature) or in an air-conditioned greenhouse (maintained at 24°C), with one plant per 4L pot.

### (2) Evaluation of Lodicule Size Phenotype and Flowering Angle

From each strain, three spikes (at a state wherein the flag leaf is still attached to the peduncle) were randomly selected, and the florets (at a stage with yellow anthers just before flowering) were collected from the central part of each spike. After removing the lemma from the florets, the lodicules were photographed using a microscope Axios Zoom v16 (Carl Zeiss, Tokyo, Japan), following the method described in the literature (Non-patent Literature 2). Based on digital images, the length, thickness, and width of the lodicules were measured using Makijaku software v1.1 (http://lbm.ab.a.u-tokyo.ac.jp/~iwata/software/makijaku/).

Meanwhile, the florets (just after flowering: the yellow anther stage) of randomly selected spikes of both the wild-type and mutant were photographed. The angle at the intersection of a line along the edge of the lemma (the glume that is farther from the spike axis) and a line along the edge of the palea (the glume that is closer to the spike axis) was measured using Makijaku software v1.1 (http://lbm.ab.a.u-tokyo.ac.jp/~iwata/software/makijaku/), and this angle was defined as the flowering angle.

### (3) Measurement of Lodicule Cells

After embedding the lodicules in a 30% acrylamide gel, the sample was cut into a section with a thickness of 150 µm using Linear Slicer PRO10 (Dosaka EM), and the section was stained using 0.01% Fluorescent Brightener 28 (Sigma). The images were taken using a laser scanning microscope (LSM700, Carl Zeiss) equipped with ZEN 2009 Light Edition CLSM software. For the measurement of lodicule cells, the number of cells corresponding to the thickness of lodicule was counted, and the cell size was calculated by dividing the thickness of lodicule by the number of cells. For each genotype, 10 samples on average were measured.

### (4) Development of Markers

For determination of SNP genotype by rough mapping, fluidigm markers were developed based on the single nucleotide polymorphisms (SNPs) of the genome sequences of the barley cultivar Sky Golden and the barley cultivar Tochinoibuki. For high-resolution gene mapping, CAPS (cleaved amplified polymorphic sequence) markers, which recognize identified SNPs around RAD markers FB0278 and FB0088, were additionally developed. These specific polymorphic sites were detected by PCR analysis, and the PCR products were sequenced according to the literature (Sakuma, S. et al., (2010) Functional & Integrative Genomics, 10:123-133.), and identified by CAPS analysis base on restriction enzymes. By comparing the two exome sequences of the Morex variety (Accession No. ERR271705) and OUH602 (Accession No. ERR271737) obtained from the DDBJ database (https://ddbj.nig.ac.jp/), 11 CAPS markers in total were developed. Additionally, InDels (insertions-deletions) markers were developed for a 22 bp deletion which was identified in the whole genome sequence of the mutant 44205. All the markers developed were used for screening the lineage of the mapping population.

### (5) Genetic Map of sof1

The F2 population of barley was developed by crossbreeding the mutant 44205 with Morex. The lodicule size was investigated using the maximized spike culture method in which spikes are treated with 2,4-dichlorophenoxyacetic acid (2,4-D) (Honda, I. et al., (2005) Physiologia Plantarum, 124:524-531.). It is known that cleistogamy perfectly matches with the size of its lodicule (non-flowering and flowering) (Nair, S. K. et al., (2010) Proceedings of the National Academy of Sciences, 107 (1):490-495.). In all the cleistogamous strains, no flowering is observed even when 2,4-D (30 ppm) was added. In contrast, the lodicules of the chasmogamous strains swelled, with the width increasing approximately 1.5 times and the thickness (in the direction perpendicular to the lemma) increasing approximately 2.3 times on average, compared to before the treatment.

For the initial mapping, 40 individuals of plants (20 individuals of WT and 20 individuals of mutant type) were selected. The genotypes of the 40 individuals of the F2 plants were determined using ddRAD-seq (Double Digest RAD-seq). In the rough mapping, genotyping was performed on 93 individuals of the F2 using fluidigm markers. The linkage map was created using AntMap version 1.2 (Iwata, H. & Ninomiya, S., (2006) Breeding Science, 56:371-377.). Next, the flowering phenotypes of the 93 individuals of the F2 were determined, and their genotypes were determined using the aforementioned CAPS markers. In the fine mapping, genotyping for segregation of the F2 between the mutant and Morex was performed using two adjacent markers, HM7H228700 and HM7H254000. The genotypes of the recombinant individuals identified with these two markers were determined using other CAPS markers developed within the genome region between HM7H228700 and HM7H254000. To accurately determine the sof1 locus, key F2 recombinants were further genotyped using InDel marker (HM7H238100-Indel), the genotypes of their F3 recombinant progeny (20 individuals) were also genotyped using InDel marker, and the lodicule size phenotypes were confirmed.

### (6) Identification of Mutation Sites in the Genome Region containing sof1 Candidate.

DNA from the leaves of fresh seedlings of the M3 generation plants of the mutant 44205 was extracted using DNeasy Plant Kit (QIAGEN). The DNA library for paired-end sequencing (150 bp) was constructed using the TruSeq DNA PCR-Free Sample Preparation Kit (Illumina). DNA sequencing was performed using HiSeqXTen platform (Illumina). The data sequence for the wild-type OUH602 was downloaded from the NCBI Short Sequence Read Archive (SRA) database (Accession: ERX5471675; Sato, K. et al., (2021) Chromosome-scale assembly of wild barley accession "OUH602". G3 (Bethesda). 27:11(10)). After trimming with Trimmomatic version 0.39 (Bolger, AM. et al., Bioinformatics, (2014) 1; 30(15):2114-2120), the sequences of the wild-type and mutant were aligned to the reference genome of the wild barley OUH602 (200720_OUH602_pseudomolecules_v1.fasta; Sato, K. et *al.,* (2021) *G3 (Bethesda),* 27;11(10)), using Bowtie2 version 2.3.5.1 (Langmead B. & Salzberg SL., (2012) Nat. Methods, 4;9(4):357-359). Variant calling of the genome sequences was performed using SAMtools version 1.10 (Li, H.et al. (2009) Bioinformatics, 15;25(16):2078-2079) (mapping score: 30, base score: 20 or higher, and a phred score threshold: 40). Since gamma-ray irradiation is expected to induce both deletion and substitutions of nucleotides, deletions that were not detected in the variant calling was identified through coverage depth analysis. The read depth of variants at each position was calculated using BCFtools (Li, H.et al. (2011) Bioinformatics, 1; 27:2987-2993). The variants were further filtered by applying a read depth threshold of 10. The positions of the mutants identified in the sof1 candidate region crossed over with the gene annotations of the Morex genome (Morex.gff.gz; Jayakodi, M. et al., (2020) Nature, 588(7837):284-289). The variant effects were predicted using SnpEff (Cingolani, P. et al., (2012) Fly (Austin), 6(2): 80-92).

### (7) Genome Sequencing of the sof1 Gene Locus.

Genomic DNA was extracted from the leaves of 2-week-old seedlings. The quality and quantity of gDNA were assessed using NanoDrop 2000 spectrophotometer (Thermo Fisher Scientific Inc.). For Sanger sequencing, the primers used for PCR amplification and sequencing were designed using NCBI/Primer-BLAST software (http://www.ncbi.nlm.nih.gov/tools/primer-blast/) and synthesized. The 20 kb genome region of the sof1 gene locus was divided into a total of five overlapping amplicons with approximately 5 kb, and at least 20 sequencing reactions were performed for each amplicon. Each amplicon was amplified using PrimeSTAR GXL DNA Polymerase (TaKaRa), a high-fidelity enzyme, according to the manufacturer's instructions. The amplification reaction, which is in total 50 µL, consisted of 100 ng of genomic DNA template, 1× PrimeSTAR GXL buffer, 200 µM of dNTP, 0.3 µM of each primer, and 2.5 units of PrimeSTAR GXL DNA polymerase. The PCR program was run for 30 cycles, each cycle consisting of "98°C for 10 seconds, 55°C or 60°C (depending on the primers) for 15 seconds, and 68°C for 4 to 5 minutes". The PCR products were purified using QIAquick PCR Purification Kit (QIAGEN), and cycle sequencing was carried out using Big Dye Terminator v3.1 technology (Applied Biosystems). Each sequencing reaction consisted of an initial denaturation at 96°C for 1 minute, followed by 25 cycles of "96°C for 10 seconds, 50°C for 5 seconds, and 60°C for 4 minutes." The reaction products were purified using Agencourt CleanSEQ System (Beckman), and analyzed using ABI Prism 3130 or 3730xL Genetic Analyzer (Applied Biosystems). The genome sequences were aligned using Seqman version 7.1.0 (DNASTAR).

### (8) Determination of the Genome Sequence of the Coding Region of sof1.

Total RNA was extracted from developing spikes at the awn primordium stage using TRIzol regent (Invitrogen) according to the manufacturer's protocol. The extracted RNA was treated with RNase-free DNase I (TAKARA BIO INC.) to remove the contamination of genomic DNA. The RNA was quantified using NanoDrop 2000 spectrophotometer (Thermo Fisher Scientific Inc.). The reverse transcription was performed using 1 µg of DNase treated-total RNA of each sample, and the first-strand cDNA was synthesized using SuperScript III Kit (Invitrogen) primed with olido-dT. The coding sequence of the sof1 gene was amplified and their sequence was determined as five amplicons using the designed primers. Each volume of 50 µL of the PCR reaction contained 20 ng of template, 1× PrimeSTAR GXL buffer, 200 µM of dNTP, 0.3 µM of each primer, and 2.5 units of PrimeSTAR GXL DNA polymerase. The PCR program consisted of 30 cycles, each cycle comprising "98°C for 10 seconds, 55°C or 60°C (depending on the primers) for 5 seconds, and 68°C for 1 to 4 minutes." The purification, sequencing and alignment of the reaction products were performed as described above.

### (9) Genome Editing via the CRISPR/Cas9-mediated Method.

The CRISPR/Cas9 system was used to induce target mutations in the Sof1 gene of barley. Three guide RNAs (gRNA, gRNA1: 5'-TGCATCCGGGAAGAAAACAG-3'/SEQ ID NO: 13, gRNA2: 5'-TGCTGCTATGAAGGAACCAG-3'/SEQ ID NO: 14, and gRNA4: 5'-ACCAGAAGAGAAGAAGGTAG-3'/SEQ ID NO: 15) were designed using WU-CRISPR website (http://crisprdb.org/wu-crispr/; Wong, N. et al., (2015) Genome Biology, 16:218). The complementary oligo DNA pairs for each gRNA sequence were inserted into the BbsI cleavage site of pHvU3-15 and pHvU3-21 (LC672614 and LC672613, respectively), constructed by replating the rice OsU6 promoter in pU6gRNA-oligo (Abe, F. et al., (2019) Cell Reports, 28:1362-1369.) with the barley HvU3 promoter. Next, the gRNA expression cassette and the scaffold sequences of pHvU3-15/21 were excised using PacI and AscI. Simultaneously, the binary vector pZH_gYSA_PubiMMCas9, containing the expression cassette for Cas9 and hygromycin phosphotransferase gene, was digested with the same restriction enzymes, and the gRNA expression cassette was introduced (Mikami, M. et al., (2015) Plant Molecular Biology, 88:561-572). The four constructed vectors, named pZH-HvU3-15: gRNA1, pZH-HvU3-15: gRNA2, pZH-HvU3-15: gRNA4, and pZH-HvU3-21: gRNA2, were used for transformation of *Agrobacterium tumefaciens* strain AGL1. They were used for transformation of barley via *Agrobacterium,* following the method described in the literature (Hisano, H. & Sato, K., (2016) Scientific Reports, 6:37505). To confirm the mutation at the target sequence of the Sof1 gene in the regenerated plants, Sanger sequencing of the PCR amplicons was performed using specific primer pairs: 5'-AGCATTGCACACTAATTCTGG-3'/SEQ ID NO: 16 and 5'-AGAAACGATTTCGGGAAGAG-3'/SEQ ID NO: 17 (for gRNA1) ; CGCTCAAGAGGATATTGCTG-3'/SEQ ID NO: 18 and 5'-TCACATGATGAAGGTTGCTG-3'/SEQ ID NO: 19 (for gRNA2); 5'-ACTCAAGTCGAACCAGTTGC-3'/SEQ ID NO: 20 and 5'-TCACATGATGAAGGTTGCTG-3'/SEQ ID NO: 21 (for gRNA4). The conditions for DNA extraction, PCR, and sequencing were according to the method described in the literature (Hisano, H. et al., (2022) Plant Biotechnology Journal, 20:37-46) .

### (10) Phylogenetic Analysis

The amino acid sequences of the protein were aligned using the default parameters of ClustalW program implemented in Molecular Evolutionary Genetics Analysis (MEGA) version 7.0 software package (Kumar, S. et al., (2016) Molecular Biology and Evolution, 33:1870-1874), and a phylogenetic tree was constructed based on neighbor-joining (NJ) supported by 1000 bootstrap replicates. The gene IDs or symbols for homologs of Arabidopsis and other species shown in the phylogenetic tree were obtained from Ensembl Plant database (http://plants.ensembl.org/Multi/Tools/Blast/). The corresponding protein IDs were based on NCBI BLASTp search (http://blast.ncbi.nlm.nih.gov/Blast.cgi).

### (11) Expression Analysis by Quantitative PCR Analysis

The spikelets were collected at 10 developmental stages from the double-ridge stage (the first structure formed from the inflorescence meristem after transition from the vegetative to reproductive growth stage, consisting of spikelet ridges and leaf ridges) to the flowering stage, using a stereomicroscope (Axio Zoom v1.6, Zeiss) following the guidelines of the literature (Kirby, E. J. M. & Appleyard, M. (1981) Cereal development guide, Cereal Unit, National Agricultural Centre. Stoneleigh, Kenilworth, Warwickshire, England.). Total RNA was extracted using TRIzol reagent (Invitrogen) from the young spikes, flower organs (including lodicules, lemma, palea, anthers, pistils, glume, and awns) at the green anther stage, the roots and leaves of 7-day-old seedlings, as well as the leaf sheaths, leaf blades, nodes, and internodes at the awn primordium stage.

The cleavage of genomic DNA, quantification, reverse transcription, and cDNA synthesis were performed as described above (refer to the section on sof1 cDNA sequence). First-strand cDNA derived from 10 ng of total RNA was used as the template for quantitative real-time PCR (qPCR). qPCR was performed using CFX96 Real-Time PCR Detection System (Bio-Rad) and THUNDERBIRD SYBR qPCR Mix Kit (Toyobo Co., Ltd.), following the protocol. The qRT-PCR data for each target gene were obtained from the PCR reactions performed for three biological replicates (three times per replicate), and the average expression levels were presented. The barley actin gene (Accession No. DN182500) was used as the endogenous control to normalize the expression levels in each sample, according to the method described in the literature (Sakuma, S. et al., (2017), Plant Physiol., 175(4):1720-1731). The relative gene expression was analyzed using the 2-ΔCt method.

### (12) RNA-seq Profiling

The plants used for RNA sequencing (RNA-seq) were grown in an air-conditioned greenhouse. The tissues of young spikes were collected from the wild-type OUH602 and the mutant 44205 at four different stages of spike development: the vegetative growth stage (VEG stage), double-ridge stage (DR stage), awn primordium stage (AP stage), and green anther stage (GA stage). At each of the four stages, six biological replicate samples were collected. Each replicate included bulk samples comprising 12 shoots from six plants at the VEG stage, 10 shoots from five plants at the DR stage, 5 young spikes from five plants at the AP stage, and 3 young spikes from three plants at the GA stage. All samples were immediately frozen using liquid nitrogen and placed in a condition at -80°C for storage until RNA extraction. RNAs from the samples at the AP stage collected from both the net house and the air-conditioned greenhouse were used also for RNA-seq. Total RNA was extracted using RNeasy Mini Kit (QIAGEN), followed by removal of genomic DNA contamination using RNA-Free DNase Set (QIAGEN). The quality of RNA samples was assessed using the Agilent 2100 Bioanalyzer (Agilent Technologies). For RNA-seq, strand-specific RNA libraries were constructed from 1 µg of total RNA from each sample using TruSeq RNA Sample Preparation Kit v2 (Illumina), which was analyzed on Illumina HiSeq X sequencing platform using two lanes (paired-end 150 bp).

After sequencing, the quality of sequence reads from all samples was evaluated using FastQC v0.11.8 (http://www.bioinformatics.babraham.ac.uk/projects/fastq c/), and adapter sequences and low-quality sequences were deleted using Trimmomatic v0.38 (Bolger, A.M., et al., (2014) Bioinformatics, 1;30(15):2114-2120). The remaining reads were mapped to the barley reference genome (Morex_pseudomolecules_v2.fasta; Jayakodi, M., et al., (2020) Nature, 588(7837):284-289) using HISAT2 v2.1.0 (Kim, D., et al., (2015) Nat. Methods, 12:357-360). Based on the gene annotation (Morex.gff; Jayakodi, M., et al., (2020) Nature, 588(7837):284-289), reads mapped to the genes were counted as expression levels using featureCounts v1.6.3 (Liao, Y., et al., (2014) Bioinformatics, 30(7):923-930), and the counted reads were further converted into transcripts per million reads (TPM) for each gene locus. Differentially expressed gene (DEG) analysis (for the samples at the awn primordium stage from the air-conditioned greenhouse and net house) was performed using Bioconductor package edgeR in the R environment (Robinson, M. D., et al., (2010) Bioinformatics, 26(1):139-140). Expression levels were normalized based on the trimmed mean of M-values (TMM) method, and P-values were calculated through an exact test based on the negative binomial distribution. The variations of gene expression were estimated based on the log2-fold change in edgeR. P-values were adjusted by the false discovery rate (FDR) based on the Benjamini-Hochberg method (Benjamini, Y., & Hochberg, Y., (1995) Journal of the Royal Statistical Society: Series B (Methodological), 57(1): 289-300). DEGs were considered significant when FDR < 0.05 and log2 > 0.5. The all annotations for DEGs were based on the results of BLASTp search (with an E-value cutoff of 10⁻⁸) using the Reciprocal Best Hits (RBH) method against the protein databases of Arabidopsis (TAIR10_-40; http://www.arabidopsis.org/) and Rice (IRGSP-1.0_predicted-protein_2020-09-09; https://rapdb.dna.affrc.go.jp/).

The heatmap of gene expression data was visualized using R package pheatmap v1.0.12. The gene expression levels at each stage were measured as the average TPM from six biological replicates and were converted to log2 (the average TPM + 1) values. For hierarchical clustering, the heatmap analysis was performed based on their expression patterns.

### (13) Detection of Splicing Event

BAM files containing the mapped reads from the RNA-seq data at the double-ridge stage, awn primordial stage, and green anther stage were merged with each genotype of the wild-type OUH602 and mutant 44205 using SAMtools version 1.9. The merged reads from the wild-type OUH602 and mutant 44205 were re-counted, and GTF files with new gene model annotations for OUH602 and mutant 44205 were created using StringTie version 2.1.4 (Pertea, M. et al., (2015) Nat. Biotechnol., 33(3): 290-295). Splice junctions were considered valid only if supported by more than 10 mapped reads. The results were visualized using ggsashimi package integrated into the R environment, following the method described in the literature (Garrido-Martin, D. et *al.,* (2018) 19(1): 67).

### (14) Statistical Analysis

All experimental data are presented as mean ± standard error (SE). Student's t-test was used to compare the phenotypes of the wild-type and sof1 mutants, as well as to examine differences in gene expression levels between the samples. The significant effects between samples were assessed based on the magnitude of the Two-tailed Student's t-test (P value < 0.05).

### B. Results

### (1) Analysis of Mutant and Wild-Type Phenotype

When the mutant 44205 and wild-type OUH602 were compared, the mutant exhibits a "super flowering" phenotype, where the lemma and the palea are more separated and the flowering angle is wider (Figures 1A and B). When observing the central spikelet, the lodicule is larger, and the mutant exhibits significantly more active cell division (Figures 1C to F). In the measurement of the floret phenotype, the mutant showed a clearly larger flowering angle compared to the wild type. In other words, the size of lodicule increased, and the lemma and the palea in the mutant were separated (Figures 1G and H, Figures 2A and B) (P<0.01, Two-tailed Student's t-test). The most important difference between the mutant and the wild type is the size of their lodicules (Figure 1H), and only based on the length of lodicules, this phenotype could be distinguished between the wild type and the mutant (Figures 2B to D). The elongation of lodicule was considered to contribute to the wider flowering angle in the mutant. Furthermore, measurements of the lodicule cells revealed that the number of cells was significantly lower in the mutant compared to the wild type (Figure 2E), corresponding to the significant increase in the length of cells in the mutant (Figure 2F). These phenotype results suggest that the elongated lodicule may be caused by cell proliferation in the mutant.

### (2) Mapping of the sof1 Gene Locus

In order to investigate the genetic factors responsible for the "super flowering" phenotype, a F2 segregating population of 93 individuals was generated by crossbreeding the mutant 44205 with Morex (non-cleistogamous). The F1 plants displayed the normal flowering angles and lodicule size, similar to their parent Morex. The F2 segregation ratio was consistent with a 3:1 Mendelian model (72 normal chasmogamous plants and 21 super flowering plants; χ² = 0.29, P > 0.05), indicating that the super flowering trait is regulated by a single recessive gene. This gene was named "sofl" (super open flowering 1).

For mapping the sof1 gene, RAD-seq analysis was performed on 40 individuals of the F2 plants. The gene was initially located between C7_77055_96891401 and C7_67011_165769003 on the chromosome 7H. Next, rough mapping using 93 individuals of the F2 confirmed that the sof1 gene locus is located in the 3.3 cM interval between fluidigm markers FB0278 and FB0088 on the chromosome 7H. Further genotyping with additional CAPS markers and the phenotypic analysis of the 93 individuals revealed that the sof1 gene locus is located within a physical region between the markers HM7H234200 and HM7H246100 with genetic distance of 1.1 cM (Figure 3A). According to the latest Morex reference genome (Jayakodi, M., et al., (2020) Nature, 588: 284-289), this physical region spans approximately 9.2 Mb and contains 118 genes, including 110 annotated genes and 8 unannotated pseudogenes.

To narrow down the physical region of the sof1 gene, recombination events were screened using a large F2 population containing 1759 individuals of sof1 mutants × Morex. Using markers HM7H228700 and HM7H254000, 99 recombinant plants were identified (Figure 3B). The length of lodicule was measured in 95 recombinant plants, excluding three plants that withered before flowering and one plant that generated smaller flower organs than the others due to a dwarf phenotype. The distribution of lodicule length fitted with a 3:1 Mendelian segregation ratio (χ² = 0.07, P > 0.05). These results were consistent with the previous data, confirming that the super-flowering phenotype is regulated by a single recessive sof1 gene, and that sof1 is located between markers HM7H234200 and HM7H246100. Further genotypic analysis and evaluation of the 13 important recombinant individuals narrowed down the region containing sof1 to a 0.37 cM interval between markers HM7H234200 and HM7H239100 (Figure 3C). The physical distance is approximately 3.1 Mb, which consisted of 43 annotated genes and 5 unannotated pseudogenes between Horvu _MOREX_7H01G234200 and Horvu _MOREX_7H01G246100 described in the literature (Jayakodi, M. et al., (2020) Nature, 588: 284-289).

### (3) Genetic Cause of Super Flowering Phenotype

In order to investigate the genetic polymorphisms between the wild-type OUH602 and the mutant 44205 within the 3.1 Mb interval, whole-genome shotgun sequencing of the mutant was performed and the result was compared with the genome sequence of the wild-type. Within this target region, only three homozygous polymorphisms were detected between the two genotypes. Among them, two 1 bp insertions were located in intergenic regions, while a 22 bp deletion was found within a gene region. The 22 bp deletion was located at position 18080 to position 18101 (with the transcription start site as 1) of the wild-type Sof1 gene genome, causing a translational frame shift in the gene in the mutant (p.Asp2646fs) (Figure 4A, B). Horvu _OUH_7H01G211300 corresponds to Horvu _MOREX_7H01G238100, a high-confidence gene in the Morex genome, and is annotated as a chromatin remodeling protein-like (Jayakodi, M. et al., (2020) Nature, 588: 284-289). For investigation of the candidate gene, In-del marker (HM7H238100-Indel) developed for the 22 bp deletion was used to genotype 13 key F2 recombinants, followed by genotype and phenotype analysis of the F3 progeny from three recombinants. As a result, it was demonstrated that In-del marker did not recombine with sof1.

### (4) Target Mutation Induction in Sof1

To support that the gene corresponding to Sof1 is Horvu _OUH_7H01G211300 in OUH602 and Horvu _MOREX_7H01G238100 in MOREX, additional mutant strains were created with the barley variety Golden Promise using CRISPR/Cas9-mediated induction of mutations. The target site of the guide RNA was directed to Horvu _GOLDEN_7H01G199000 gene (near the 22bp deletion in the mutant), the ortholog of Horvu _MOREX_7H01G238100 in the Golden Promise genome (Figure 5A). After genotyping and sequencing of the transgenic plants, an independent mutation with a 5bp deletion (sof1-2) was obtained. This corresponded to the introduction of a stop codon that leads to premature translation termination, which therefore can be considered as a loss-of-function genetic mutation allele (Figure 5A). Golden Promise holds the cleistogamous allele (clyl.b) at the clyl gene locus and forms small lodicules, but the generated genome-edited strains also hold the cleistogamous allele, derived from this cultivar, which potentially interferes with the effect of the sof1 mutation. To eliminate the potential negative effect of clyl.b on the development of lodicule, the sof1-2 T0 strains were crossbred with non-cleistogamous barley varieties Sv73528 and Adorra, the F3 population genotyped as sof1-2sof1-2Cly1_was obtained, and the lodicule phenotype was determined (Figure 5B). The result was that the lodicule length of the sof1-2 mutants (T2) was significantly longer compared to Sv73528 and Adorra (Figure 6).

Compared to Sv73528 and Adorra, the F3 plants had significantly longer lodicules and showed greater flowering angles (Figure 7), but there was no big difference in lodicule depth and width. These results suggested that the elongated lodicules in the sof1-2 mutants were caused by the cleavage of Horvu _GOLDEN_7H01G199000, and that Horvu_MOREX_7H01G238100 was considered to be the functional gene for Sof1. Therefore, it was concluded that the 22-bp deletion in Horvu_MOREX_7H01G238100 is the cause of the super-flowering phenotype of the sof1 mutants.

### (5) Structural Features of the Sof1 Gene

Sof1 is the wild-type allele of sof1. The Sof1 gene has 20720 bp from the start codon to the stop codon (SEQ ID NO: 3), with the full-length CDS being 10326 bp (SEQ ID NO: 1) (Figure 4A), which encodes a protein with 3441 amino acids (SOF1) that is predicted to have two SNF2 family domains (Figure 4B). One is an ATPase domain composed of SNF2_N (N-terminal domain of the SNF2 family) and Helicase_C (Helicase conserved C-terminal domain), and the other is SnAC (Snf2 ATP coupling) domain (Figure 4B). The ATPase domain and SnAC domain are located at the N-terminus of SOF1, but no conserved domains are found in the C-terminal region. Compared to the wild-type, the sof1 mutant contains a 22 bp deletion (Figure 4A), which causes a frameshift mutation (D2659Efs), introducing a stop codon at the nucleotide corresponding to the 2677th amino acid of the C-terminal region, resulting in a truncated protein composed of 2676 amino acids that lacks the last 765 amino acids (approximately 22% of the full SOF1 protein) (Figure 4B). The truncated protein was believed to lose its biological function in the mutant. SNF2 is an ATP-dependent chromatin remodeling factor that regulates many aspects of DNA events, including transcription, replication, homologous recombination, and DNA repair. To investigate the function of SOF1, phylogenetic analysis of SOF1 homologs was performed. Based on BLASTp search in plant varieties, SOF1 homologs were found in both monocotyledons and dicotyledons. For use in phylogenetic analysis, highly-ranked homologs with high identity to SOF1 were further selected from each classified group. The other three Arabidopsis SWI/SNF subfamily members were included in order to infer the class to which the homologous proteins belong. The phylogenetic tree showed that all SOF1 homologs clustered within the SWI/SNF subfamily, suggesting that SOF1 belongs to this SWI/SNF subfamily (SWI/SNF ATPase), and that these sequences are orthologous (Figure 8). The SWI/SNF ATPase has the function of activating chromatin and turning ON its genes. In this SWI/SNF cluster, SOF1 and Arabidopsis SPLAYED (SYD) proteins were closely related to each other (Figure 8). SYD of Arabidopsis acts on several of the downstream bHLH genes via multiple MADS box genes, thereby suppressing petal enlargement. Therefore, mutations in SYD result in larger petals. In *Poaceae,* the organ homologous to petal is lodicule. Therefore, the gene regulatory network originating from SYD can be used as one model for the lodicule enlargement (super flowering) in the barley sof1 mutation. On the other hand, the homolog HORVU_MOREX_6H01G041600.1 is annotated as an ATP-dependent helicase family protein in the Morex genome. This shows a close relationship with Arabidopsis BRAMHA (BRM), and it was indicated that it contains a potential bromodomain at the C-terminus based on NCBI conserved domain database (Marchler-Baur, A. et al., (2017) Nucleic Acids Res., 4;45(D1): D200-D203). It was suggested that HORVU_MOREX_6H01G041600.1 is a paralog of SOF1 in barley. On the other hand, OsSYD (XP_015642458.1) is reported to be one of the SYD orthologs based on phylogenetic analysis and as the SYD ortholog in *Oryza sativa* ssp *japonica* (Su, Y. et al., (2006) The Plant Journal, 46(4): 685-699, Hu, Y. et al., (2013) Plant physiology and biochemistry, 70: 33-42). These results suggest that SOF1 is an ortholog of Arabidopsis SYD. SOF1 (and SYD) is conserved in both monocotyledons and dicotyledons, suggesting that these SOF1 orthologs are derived from a common ancestor and have been evolutionarily conserved (Figure 8). Therefore, Sof1 codes for a chromatin remodeling ATPase protein, and its function may be similar to that of Arabidopsis SYD and OsSYD.

### (6) Profiling of Transcript Levels of Sof1

Transcriptional levels of Sof1 were examined in the entire spike, individual reproductive organs at the different developmental stages of the spike, and various vegetative organs (Figure 9). qRT-PCR profiling revealed that this gene is broadly transcribed throughout the spike development stages in both the wild-type and mutant. The transcription of this enzyme could be detected in 6 floral organs, leaf sheaths, leaf blades, nodes, and internodes at the green anther stage, but the young spikes at the same awn primordium stage was also low. The transcript levels in the mutant were significantly lower in the young spikes and reproductive organs compared to those of the wild-type (Figure 9). Notably, the transcription levels of this gene significantly decreased in the mutant from the awn primordium stage to flowering, and were clearly decreased in the lodicule as well (P < 0.01, Figure 9). The transcript level analysis suggested that this gene may play a role between vegetative growth and reproductive development.

### (7) Estimated Transcriptional regulation by Sof1

In order to reveal the impact of the 22 bp deletion in Sof1 using transcriptome analysis, RNA-seq analysis was performed on the wild-type OUH602 and the mutant 44205. The analysis of immature spikes (awn primordium stage) grown in both the air-conditioned greenhouse (with natural light at 22°C) and the net house revealed a total of 2412 and 1371 differentially expressed genes (DEG) (FDR < 0.05 and | log2 fold change | > 0.5), respectively, between the genotypes under the two growth conditions. Furthermore, the 674 common DEGs were comprised of 391 (58%) genes with induced expression and 283 (42%) genes with suppressed expression in the mutant compared to the wild type. The decrease in Sof1 expression in the mutant was confirmed also by RNA-seq analysis, which were consistent with the Sof1 expression profile obtained through qRT-PCR.

Among the genes with significantly suppressed expression, six flower homeotic genes were identified. These include B-class genes (AP3-like, Horvu_MOREX_7H01G536500 and PI-like, Horvu_MOREX_1H01G402000), C-class genes (AG-like, Horvu_MOREX_3H01G171300), E-class (AGL6-like, Horvu_MOREX_6H01G395900 and SEP-like, Horvu_MOREX_7H01G328200), and SVP-like gene (Horvu_MOREX_4H01G454700), which were homologous to OsMADS16, OsMADS4, OsMADS3, OsMADS6, and OsMADS6 in rice, respectively. In particular, the genes OsMADS16 (Nagasawa, N. et al., (2003), Development, 130(4): 705-18; Yoshida, H. et al., (2007), Plant Biotechnology Journal, 5(6): 835-846), OsMADS3 (Yamaguchi, Y. et al., (2006), The Plant Cell, 18(1): 15-28; Dreni, L. et al., (2011), The Plant Cell, 23(8): 2850-2863), OsMADS6 (Li, H. et al., (2010), Cell research, 20(3): 299-313), and OsMADS7 (Cui, B. et al., (2010), Plant J., 61(5): 767-781) have been reported to be involved in the development of the lodicules via rice mutants.

### (8) Estimation of Pollination Efficiency with Different Flowering Ability

### (15) Estimation of Pollination Efficiency

In order to estimate the pollination effect, cytoplasmic male sterility strains of two chasmogamous varieties (Adorra, Sv73528) and one cleistogamous variety (Kawasaigoku) were used. During the flowering period of the potted plants, the glumes were physically opened, the awns were fixed with tape, pollinated artificially, and let the grains matured. The pollination effect was then estimated based on the number of grains set. As a result, a significant pollination effect (increase in the number of grains set) of more than five times was observed in the chasmogamous varieties Adorra and Sv735108. The effect was also evident in the cleistogamous variety "Kawasaigoku" (Table 1).

**[Table 1]**

| Difference in the Number of Grains S et (%) by Flower Opening Treatment | | | |
|---|---|---|---|
| Chasmogamous/ Cleistogamous Type | Chasmogamous Type | Chasmogamous type | Cleistogamous type |
| Variety | Adorra | Sv73528 | Kawasaigoku |
| Non-treated Flower Opening Treatment | 8.5 | 12.8 | 7.5 |
| | 54.5 | 60.2 | 12.1 |

No grain set was observed in the spikes of the male sterile strains that cannot be pollinated, confirming that they were not fertilized by pollen from other individuals or through any means other than the pollination treatment. In the chasmogamous mutants that can be pollinated, a significant increase in the number of grains set was observed due to the improvement in pollination rate.

### (8) Identification and Analysis of Wheat Homologous Genes

From the genomic information of the wheat variety Chinese Spring, the homologous gene sequences in wheat were obtained. The base sequences of the cDNA of each identified wheat Sof1 gene are shown in SEQ ID NOs: 4, 7, and 10; the base sequences of the genomic DNA are shown in SEQ ID NOs: 6, 9, 12; and the amino acid sequences of the protein coded by these DNAs are shown in SEQ ID NOs: 5, 8, 11. Similar to barley Sof1, it was found that Sof1 is strongly expressed in various organs such as roots, leaves, and spikes in wheat (Figure 10). Furthermore, based on the TILLING method, multiple mutant lineages of the wheat Sof1 gene were successfully screened in the wheat varieties Kronos and Cadenza.

### [Industrial Applicability]

As described above, according to the present invention, a *Triticeae* plant with improved flowering ability may be produced. Because this invention enables significant enhancement of the efficiency of developing new *Triticeae* varieties, it would contribute to the improvement of grain productivity in the field of agriculture.

## Claims

1. A method for producing a *Triticeae* plant with improved flowering ability, which comprises artificially suppressing the function of the following endogenous gene (a) or (b) in a *Triticeae* plant:
(a) an endogenous gene encoding a protein consisting of an amino acid sequence of SEQ ID NOs: 2, 5, 8 or 11;
(b) an endogenous gene encoding a protein consisting of an amino acid sequence having at least 80% homology to the amino acid sequence of SEQ ID NOs: 2, 5, 8 or 11.

2. A *Triticeae* plant with improved flowering ability, wherein the function of the following endogenous gene (a) or (b) is artificially suppressed:
(a) an endogenous gene encoding a protein consisting of an amino acid sequence of SEQ ID NOs: 2, 5, 8 or 11;
(b) an endogenous gene encoding a protein consisting of an amino acid sequence having at least 80% homology to the amino acid sequence of SEQ ID NOs: 2, 5, 8 or 11.

3. A method for evaluating flowering ability of a *Triticeae* plant, which comprises analyzing a base sequence of the following endogenous gene (a) or (b) or a regulatory region thereof in a *Triticeae* plant:
(a) an endogenous gene encoding a protein consisting of an amino acid sequence of SEQ ID NOs: 2, 5, 8 or 11;
(b) an endogenous gene encoding a protein consisting of an amino acid sequence having at least 80% homology to the amino acid sequence of SEQ ID NOs: 2, 5, 8 or 11.

4. A method for evaluating flowering ability of a *Triticeae* plant, which comprises analyzing the expression of the following endogenous gene (a) or (b) in a *Triticeae* plant:
(a) an endogenous gene encoding a protein consisting of an amino acid sequence of SEQ ID NOs: 2, 5, 8 or 11;
(b) an endogenous gene encoding a protein consisting of an amino acid sequence having at least 80% homology to the amino acid sequence of SEQ ID NOs: 2, 5, 8 or 11.

5. A method for producing a *Triticeae* plant with improved flowering ability, which comprises crossbreeding the *Triticeae* plant of claim 2 with another arbitrary *Triticeae* plant.
